# EUROPEAN PATENT APPLICATION

(11) **EP 1 110 575 A2**
(43) Date of publication of application: **27.06.2001**
(21) Application number: 00311421.2
(22) Date of filing: 20.12.2000
(51) Int. Cl.: A61M 25/06

(54) **Catheter with guiding needle avoiding temporary exit of bodily fluid**

(30) Priority: 21.12.1999 US 469738
(71) Applicant: ETHICON, INC., Somerville New Jersey 08876 (US)
(72) Inventor: Chang, Joseph J., Irving, Texas 75063 (US)
(74) Representative: Mercer, Christopher Paul

(57) **Abstract**

A catheter unit comprising a needle, an elongated blunting member coupled to a flash chamber and to a safety member, the blunting member having a blunt distal tip and an opened proximal end for allowing blood to flow generally directly to a porous member at the proximal end of the blunting member. The blunting member has a hollow lumen therebetween extending longitudinally through the needle such that the blunting member is disposed coaxially within the bore of the needle.

## Description

### BACKGROUND OF THE INVENTION

### Field of the Invention

The present invention relates generally to medical devices, and more particularly to a needle blunting apparatus used in an intravascular assembly such as a catheter.

### Description of Related Art

Intravascular assemblies such as catheter assemblies are generally used for passing fluids between a device such as a syringe or a drip to or from body lumens such as veins or arteries, or other internal target sites. A catheter assembly usually includes a hub, a catheter, and a needle. An eyelet ring is typically inserted into the catheter. The catheter, together with the eyelet ring, is then inserted into an opening in the nose of the hub and is secured to the hub by press fitting the eyelet ring within the nose of the hub. A needle is then inserted into the catheter. A sharp tip of the needle is used for piercing a body lumen so that access may be gained into the body lumen by the catheter and the needle. Once the catheter and the needle are located within the body lumen, the needle is removed. A syringe or a pipe of a drip is then attached to the hub so that fluids may be passed through the hub and the catheter between the drip or the syringe and the body lumen. The hub is typically made of materials that provide sufficient rigidity thereto and the catheter is usually made of a material which is flexible.

Despite advances made regarding catheters, the escape of blood or other bodily fluids during use remains a significant problem, potentially exposing a healthcare worker or another person to blood-borne pathogens. In view of the potential exposure risk of contacting blood born pathogens such as HIV and hepatitis, there exists a need to provide catheters that reduce this risk. Although existing devices are capable of reducing the risk that a person will contact blood-borne pathogens through inadvertent needle trauma, none of these prior devices or apparatuses are universally usable in connection with all types of catheters. Accordingly, there remains a need for the development of additional needle blunting devices and/or apparatus for preventing or reducing the risk of exposure to blood or other bodily fluids due to fluid escaping from the catheter.

### SUMMARY OF THE INVENTION

An apparatus is disclosed that includes an elongated blunting member coupled to a flash chamber and to a safety member, the blunting member has a blunt distal tip and an opened proximal end for allowing blood to flow generally directly to a porous member at the proximal end of the blunting member. The blunting member has a hollow lumen therebetween extending longitudinally through the needle, the blunting member being disposed coaxially within the bore of the needle. Additional features, embodiments, and benefits will be evident in view of the figures and detailed description presented herein.

### BRIEF DESCRIPTION OF THE DRAWINGS

Features, aspects, and advantages of the invention will become more thoroughly apparent from the following detailed description, appended claims, and accompanying drawings in which:
**Figure 1** is a cross-sectional view of an assembly incorporating a needle blunting apparatus according to an embodiment of the invention.
**Figure 2** is a cross-sectional view of the needle component, the blunting member, and a porous member of the assembly of **Figure 1**.
**Figure 3** is a cross-sectional view of a flash chamber used in the assembly of **Figure 1**.
**Figure 4** is a cross-sectional view of the blunting apparatus of the assembly of **Figure 1**.
**Figure 5** is a partial longitudinal sectional view of the blunting apparatus of assembly of **Figure 1**.
**Figure 6** is a partial longitudinal sectional view of the needle component of the assembly shown in **Figures 1-3** having the needle blunting apparatus of the invention in its advanced "blunting" position.
**Figures 7** through **9** are a step-wise illustration of one method of using the assembly described herein.
**Figure 10** is a partial longitudinal sectional view showing the needle blunting apparatus.

### DETAILED DESCRIPTION OF THE INVENTION

The following detailed description and the accompanying drawings are provided for the purpose of describing and illustrating presently preferred embodiments of the invention only, and are not intended to limit the scope of the invention in any way.

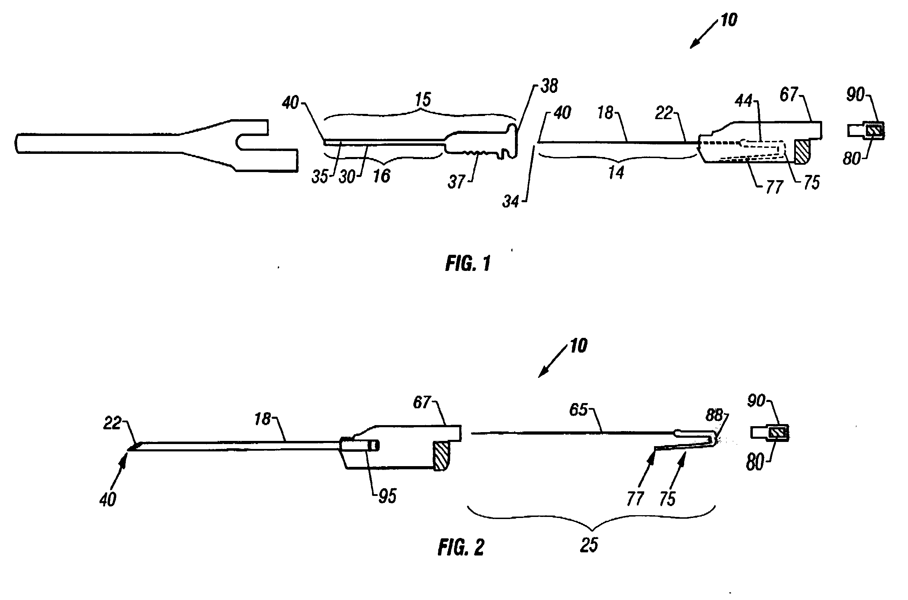

With reference to one embodiment of the invention shown in **Figures 1-2**, there is provided assembly 10 that may be used to facilitate percutaneous insertion of an intravascular cannula, tube, and catheter. **Figure 2** shows assembly 10 generally comprises introducer 15, needle 18, needle blunting assembly 25, and protective outer sheath 16 (**Figure 1**).

Introducer 15 comprises an elongated tubular cannula 30 with a hollow lumen 35 extending longitudinally through cannula 30. A tapered distal tip is formed on the distal end of the cannula 30 to facilitate insertion and advancement of the cannula through skin, connective tissue, or a blood vessel wall.

Assembly 10 also includes housing 67 coupled to needle blunting assembly 25. At the proximal end of housing 67, member 90 is coupled thereto. Member 90 has a lower cylindrical portion that has an outside diameter that is smaller than the inner diameter of housing 67. Member 90 has a hollow lumen therethrough allowing porous member 80 to be positioned within member 90.

In the preferred embodiment, porous member 80 has a porosity approximately in the-range of 35% to 55% and is preferably in the range of approximately 45%. This porosity prevents blood or other bodily fluids from exiting housing 67 and contacting a person such as a healthcare worker for a certain time period such as thirty seconds. This time period should adequately protect a health worker such that bodily fluids do not fill the flash chamber 37 wherein pressure build-up results in reverse-fluid pressure to the introducer 15.

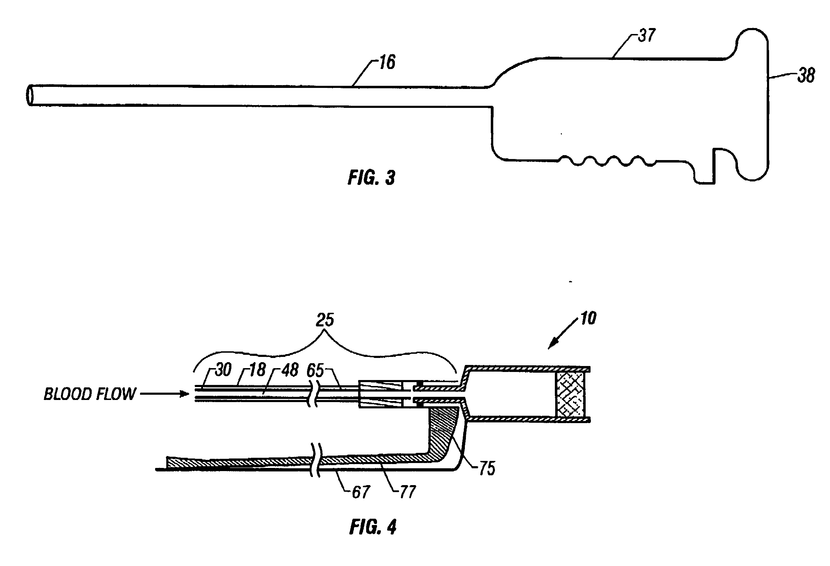

Porous member 80 may be comprised of materials such as cotton high-density polyethylene (HDPE) or ultra-high-molecular-weight polyethylene (UHMWPE). Elongated rigid needle 18 is formed of material such as stainless steel hypotubing and has a beveled or otherwise sharpened distal tip 40. As shown in **Figure 3**, a hollow bore 22 extends longitudinally through needle 18. A transparent flash chamber housing 37 is coupled to the proximal end of the elongated rigid needle 18. A hollow flash chamber bore 38 extends longitudinally through the proximal flash chamber housing 37. Such longitudinal flash chamber bore 38 has a substantially cylindrical proximal inner wall of substantially continuous diameter and a narrowed or tapered distal inner wall 60. The hollow inner bore 38 of flash chamber housing 37 is continuous with and connected to the hollow bore 22 of needle 18 as shown in **Figure 1** wherein these elements coaxially nestled together.

**Figure 4** shows needle blunting apparatus 25 of assembly 10 including an elongated tubular blunting member 65 preferably formed of rigid material such as stainless steel hypotubing. Blunting member 65 and needle 30 may form a single integral piece or they may be separate and secured together by methods known in the art. One such method involves blunting member 65 having a smaller outer diameter in comparison to the inner diameter of needle 30 such that blunting member 65 comfortably slides into needle 30 forming a secure member to pierce the skin or connective tissue of a human.

Blunting member 65 has a hollow lumen 48 that extends longitudinally therethrough. Blunting member 65 is coupled to or otherwise associated with securing member 75 such as a hook to anchor or hold the blunting member in its position. Other suitable configurations also may be used. Securing member 75 may be comprised of flexible or elastic material such as polymers including plastic. Securing member 75 has a longitudinal portion 77 that extends toward the distal end of blunting member 65. The longitudinal portion of securing portion 75 is positioned to press against the inner wall of housing 67 for blunting member 65.

Given the above description, the flow of bodily fluids through assembly 10 may occur generally in the following fashion. Needle 18 pierces the skin of a patient and enters a vessel such as a blood vessel. Blood or other bodily fluids enters the hollow cavity of needle 18 and moves generally in the direction of blunting member 65. Thereafter, the bodily fluid enters flash chamber 37. Flash chamber 37 generally serves the purpose of containing bodily fluids. As flash chamber 37 fills with bodily fluid, the bodily fluid may contact porous member 80. While pressure builds in assembly 10, the bodily fluid follows a tortuous path through the pores or unobstructed paths in porous member 80. Porous member 80 prevents bodily fluid from exiting porous member 80 for a certain time period by absorbing this fluid. For example, porous member 80 may prevent bodily fluids from escaping up to thirty seconds after flash chamber 37 is completely filled.

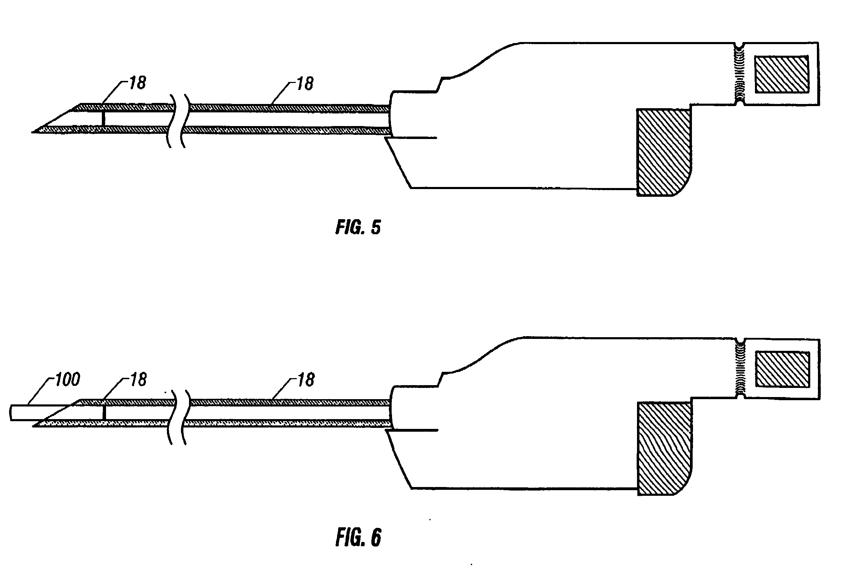

It will be appreciated from **Figures 1** and **2** that the introducer 15, the needle component 20, and the blunting apparatus 25 are initially disposed in a coaxially nested arrangement wherein needle 18 extends coaxially through the lumen 35 of cannula 30. As noted above, blunting member 65 extends through a portion of the bore 22 of needle 18 such that the blunt distal tip 100 of blunting member 65 is located within the bore 22 of needle 18 a spaced distance X, proximal to its sharpened distal tip 100. Thereafter, pushing blunting assembly 25 in the direction of needle 18 will cause the blunting assembly 25 to be advanced in the distal direction as shown in **Figure 6**, while pulling blunting assembly 25 away from needle 18 will cause the blunting assembly 25 to be retracted in the proximal direction as shown in **Figure 5**. It will be appreciated that, when the blunting apparatus 25 is in its proximally retracted "non-blunting" position the blunt distal tip 100 resides within lumen 22 of the elongated rigid needle 18, a spaced distance X₁ from the distal tip 100 thereof. However, when the blunting apparatus 25 is moved to its distally advanced "blunting" position, the blunt distal tip 100 of the tubular member 65 will extend out of and beyond the sharp distal tip 100 of the elongate rigid needle 18 by a distance X₂. Such protrusion of the blunt distal tip 100 of the tubular member 65 beyond the sharpened distal tip 100 of the elongated rigid needle 18 essentially prevents the sharpened distal tip 100 of the elongate rigid needle causing trauma to, or puncturing, skin, or other tissue.

It will be appreciated that engagement member 52 of the blunting apparatus 25 may be formed or configured in various different ways, without departing from its intended functions, including the function of facilitating movement of the blunting apparatus 25 between its proximally retracted "non-blunting" position as shown in **Figure 5**, and its distally extended "blunting" position as shown in **Figure 6**.

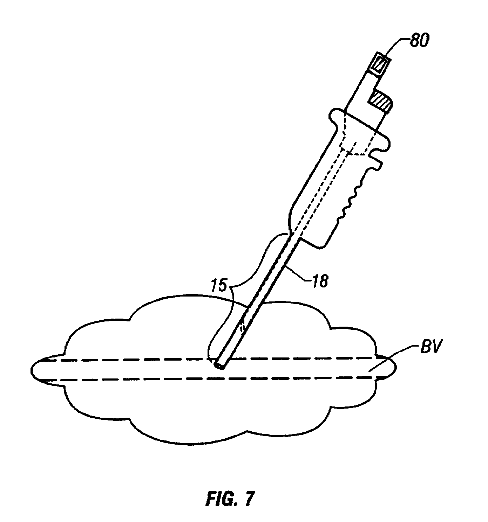

**Figures 7** though **10** show one embodiment of the invention in which a catheter is inserted into a patient. With reference to **Figures 7** through **10** the blunting apparatus 25 is initially retracted to its "non-blunting" position as shown in **Figure 5**. Needle 18 having introducer 15 disposed thereon is then percutaneously inserted into a blood vessel BV, as shown in **Figure 7**. The presence of blood in the flash chamber provides an indication that blood vessel BV has been entered.

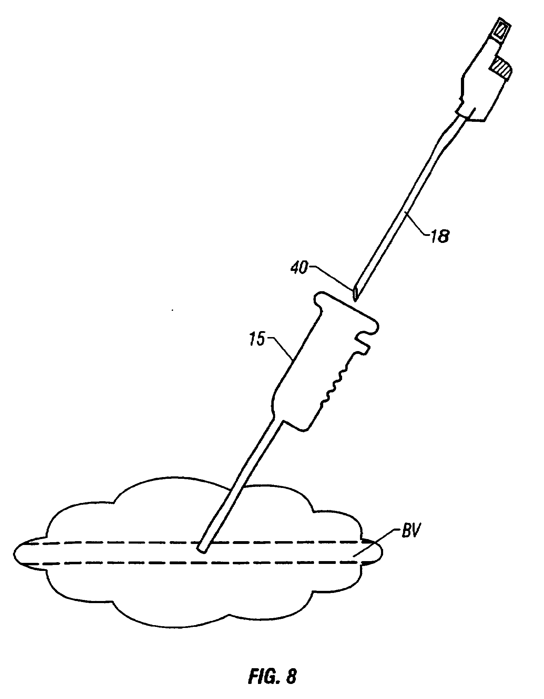

Thereafter, the blunting apparatus 25 is advanced to its distally advanced "blunting position" as shown in **Figure 6** and the needle 18 is withdrawn (**Figure 8**). Because the blunt distal tip 100 of the blunting member 65 of the blunting apparatus 25 extends to X₂, a distance that is beyond the beveled or sharpened distal tip 40 of the needle 18, the needle 18 is thereby rendered incapable of puncturing or causing trauma to the user or other persons who have occasion to handle a used needle 18.

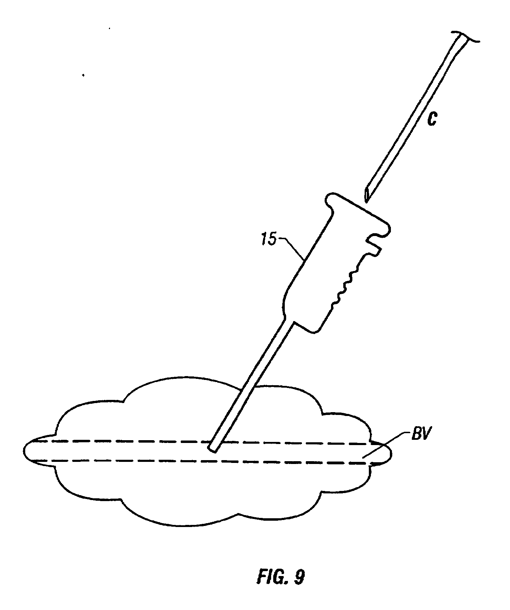

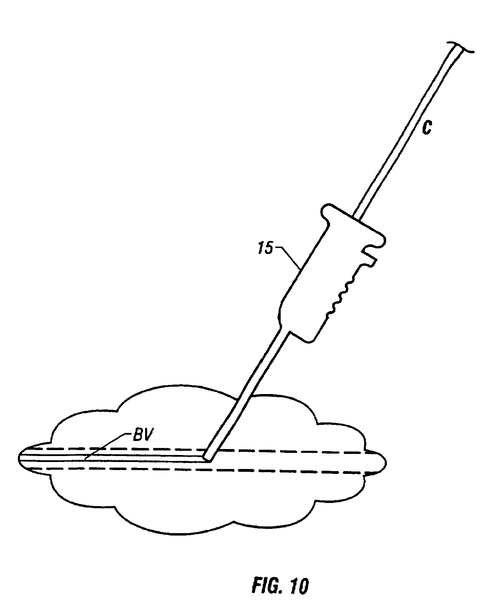

After the needle 18 and blunting apparatus 25 have been removed and discarded, a tubular catheter C is advanced through the introducer 15, as shown in **Figure 9**. **Figure 10** shows the introducer 15 is proximally withdrawn, leaving the catheter C within the blood vessel BV. After introducer 15 has been withdrawn, it resides about an exteriorized portion of the catheter C.

In the preceding detailed description, the invention is described with reference to specific embodiments thereof. It will, however, be evident that various modifications and changes may be made thereto without departing from the broader spirit and scope of the invention as set forth in the claims. The specification and drawings are, accordingly, to be regarded in an illustrative rather than a restrictive sense.

## Claims

1. A catheter unit comprising:
a needle; and
an elongated blunting member coupled to a flash chamber and to a safety member, the blunting member having a blunt distal tip and an opened proximal end for allowing blood to flow generally directly to a porous member seated within a member at the proximal end of the blunting member;
the blunting member having a hollow lumen therebetween extending longitudinally through the blunting member, the blunting member being disposed coaxially within the bore of the needle.

2. The catheter unit of claim 1, wherein the porous member is functionally open allowing fluid from a patient to exit the catheter unit after thirty seconds of blood entering the flash chamber.

3. The catheter unit of claim 1, wherein the flash chamber has a proximal end and a distal end and a porous member is attached to distal end of the flash chamber.

4. The catheter unit of claim 3, wherein the porous member is removable.

5. The catheter unit of claim 3, wherein the porous member is approximately in the range of 35% to 55% of porosity.

6. An intravascular assembly, the assembly comprising:
a tubular introducer sheath having a proximal end, a distal end and a hollow lumen extending longitudinally therethrough;
a needle having a sharpened distal tip and a hollow bore extending longitudinally therethrough, the needle being disposed coaxially within the lumen of the introducer sheath;
an elongated blunting member having a hollow lumen extending longitudinally therethrough without apertures and having an opened proximal end and a blunt distal tip, the elongated blunting member being disposed coaxially within the bore of the needle;
the blunting member being axially moveable from a non-blunting position wherein the blunt distal tip of the blunting member is positioned within the bore of the needle a spaced distance proximal to the sharpened distal tip of the needle, to a distally advanced blunting position wherein the blunt distal tip of the blunting member protrudes out of and beyond the sharpened distal tip of the needle.

7. The assembly of claim 6, wherein an at least partially transparent flash chamber is formed on the proximal end of the blunting member; and, wherein the blunting apparatus further comprises:
a lumen which extends longitudinally through the blunting member;
the assembly being thereby operative such that when the distal end of the needle enters a vessel, such that fluid enters the bore of the needle and passes through the needle and then enters the lumen of the blunting member and exits the blunting member by entering the flash chamber, such that the presence of blood within the flash chamber is visible through at least a transparent portion of the flash chamber and whereby the fluid may contact a porous member which is coupled to a housing for the blunting member.

8. The catheter unit of claim 6, wherein the porous member is functionally open allowing fluid from a patient to exit the catheter unit after thirty seconds of blood entering the flash chamber.

9. A catheter comprising
a needle;
an elongated blunting member coupled to the needle and to a stopper, the blunting member causing blood to flow generally directly to a stopper, the stopper is coupled to a chamber.

10. The catheter of claim 9, wherein the stopper is porous.

11. The catheter of claim 9, wherein the stopper is removable.

12. The catheter of claim 9, wherein the stopper has porosity approximately in the range of 35% to 55%.

13. The catheter unit of claim 9, wherein the porous member is functionally open allowing fluid from a patient to exit the catheter unit after thirty seconds of blood entering the flash chamber.
